# EUROPÄISCHE PATENTSCHRIFT

(11) **EP 2 051 745 B1**
(45) Veröffentlichungstag und Bekanntmachung des Hinweises auf die Patenterteilung: **09.03.2011**
(21) Anmeldenummer: 07724798.9
(22) Anmeldetag: 02.05.2007
(51) Int. Cl.: A61L 2/28, A61B 19/00, B08B 9/00

(54) **Vorrichtung zur Überprüfung des Reinigungs- und Desinfektionsergebnisses bei insbesondere in Waschautomaten gereinigten diagnostischen und chirurgischen Instrumenten**
Device for verifying the cleaning and disinfection result of diagnostic and surgical instruments which are especially cleaned in automatic washers
Dispositif destiné à vérifier les résultats de nettoyage et de désinfection d'instruments de diagnostic et chirurgicaux nettoyés notamment dans des machines à laver automatiques

(30) Priorität: 14.08.2006 AT 6142006 U
(43) Veröffentlichungstag der Anmeldung: 29.04.2009
(73) Patentinhaber: Helmut, Pach, 6521 Fliess (AT)
(72) Erfinder: Helmut, Pach, 6521 Fliess (AT)
(74) Vertreter: Grape & Schwarzensteiner
(86) Internationale Anmeldenummer: PCT/EP2007/003872
(87) Internationale Veröffentlichungsnummer: WO 2008/019715

(56) Entgegenhaltungen:
- EP-A1- 1 488 757
- EP-A1- 1 704 872
- EP-A1- 1 886 622
- WO-A-01/45755
- WO-A1-2005/053755
- DE-U1- 20 108 346
- US-A- 5 872 004
- US-A- 5 872 004
- US-A1- 2003 012 688
- US-A1- 2003 215 923

## Beschreibung

Die Erfindung betrifft eine Vorrichtung zur Überprüfung des Reinigungs- und Desinfektionsergebnisses bei insbesondere in Waschautomaten gereinigten diagnostischen und/oder chirurgischen Instrumenten mit gehäuseförmigen und kanülenförmigen Bauteilen, wie Endoskopen.

Mit solchen Vorrichtungen soll überprüft werden, ob gereinigte Instrumente der vorgenannten Art nach einem nach der Anwendung vorzunehmenden Reinigungsvorgang sowohl an der Außenfläche als auch in Hohlräumen des Instrumentes frei von Körperfluiden sind, mit welchen diese bei der Anwendung in Kontakt gebracht wurden, wie beispielsweise Blut, Gewebsflüssigkeit oder Sekrete, die mit Erregern jeglicher Art kontaminiert sein können.

Zu diesem Zweck ist aus der DE 201 08 346 U1 ein Prüfsystem bekannt, das aus einem ersten und einem zweiten Hohlkörper besteht, die miteinander verbindbar sind, wobei im Verbindungsbereich der beiden Hohlkörper ein Prüfkörper einbringbar ist. Dieses Prüfsystem ist nur zur Überprüfung der Reinigungsleistung eines Waschautomaten im Zusammenhang mit kanülenförmigen Bauteilen eines diagnostischen und/oder chirurgischen Instrumentes verwendbar und darüber hinaus aufgrund seiner Ausgestaltung nicht in der Lage die Gesamtsituation in einem diagnostischen und/oder chirurgischen Instrument zu simulieren.

Weiters sind Prüfsysteme bekannt, bei denen das Durchflussvolumen bzw. der sich bei einer Verengung des innenliegenden Hohlraums des kanülenförmigen Arbeitsteiles aufbauende Staudruck gemessen wird und anhand des Messergebnisses Rückschlüsse auf die Restverschmutzung des kanülenförmigen Arbeitsteils bzw. die Reinigungsleistung eines Waschautomaten gezogen werden. Wieder lässt sich mit diesem System nicht die Gesamtsituation in einem diagnostischen und/oder chirurgischen Instrument simulieren. Darüber hinaus ist das erhaltene Ergebnis lediglich relativ und kann nichts über die Art der Verschmutzung aussagen.

Die US 2003/0012688 A1, US 2003/0215923 A1 und EP 1 488 757 A1 beschreiben Vorrichtungen und Verfahren zur Bestimmung bzw. Überprüfung der Reinigungs- und Desinfektionswirkung von Waschautomaten von klinischen Instrumenten, wie Endoskopen. Vorrichtungen und Verfahren können dazu verwendet werden, die Wirksamkeit der Reinigung bzw. Desinfektion in dem Arbeitsteil eines Endoskopes einzuschätzen.

Die WO 2005/053755 A1 beschreibt eine Vorrichtung zur Überprüfung des Reinigungsergebnisses bei einem in Waschautomaten gereinigten Endoskop, die als eine Attrappe des Endoskops ausgebildet ist. Es handelt sich dabei gegebenenfalls um mehrere, den verschiedenen Teilen eines Endoskops entsprechende Prüfkörper bzw. Prüfschlauch, wie einem Bedienteil und einem Arbeitsteil. Die Prüfkörper sind mit Prüfkammern zur Anordnung eines Prüfelementes mit Testverschmutzung ausgestattet, wie einer zweiteilig ausgestalteten Prüfkammer.

In der WO 01/45755 ist ferner eine Vorrichtung beschrieben, mit welcher die Reinigungswirkung an Oberflächen überprüft werden kann.

Mit anderen Worten, die aus dem Stand der Technik bekannten Vorrichtungen weisen generell den Nachteil auf, dass sich mit ihnen lediglich das Reinigungs- und Desinfektionsergebnis bezüglich des kanülenförmigen Bauteiles überprüfen lässt. Hinsichtlich des gehäuseförmigen Bauteiles lässt sich mit den bekannten Vorrichtungen lediglich überprüfen, ob dessen Außenfläche den Anforderungen entsprechend gereinigt wurde. Dies stellt insbesondere bei einteiligen Instrumenten, bei denen das flexible kanülenförmige Bauteil unlösbar mit dem gehäuseförmigen Bauteil verbunden ist, ein nicht zu unterschätzendes Risiko einer insbesondere im gehäuseförmigen Bauteil bzw. im Verbindungsbereich zwischen diesem und dem kanülenförmigen Bauteil verbleibenden Restverschmutzung dar.

Der Erfindung liegt daher die Aufgabe zugrunde, eine verbesserte Vorrichtung zur Überprüfung des Reinigungs- und Desinfektionsergebnisses bei insbesondere in Waschautomaten gereinigten und/oder desinfizierten diagnostischen und/oder chirurgischen Instrumenten mit gehäuseförmigen und/oder kanülenförmigen Bauteilen zu schaffen, mit der die zuvor beschriebenen Nachteile vermieden werden können und die eine umfassende Überprüfung des Reinigungs- und Desinfektionsergebnisses eines solchen Instrumentes ermöglicht.

Die Erfindung löst diese Aufgabe mit einer Vorrichtung gemäß Anspruch 1 vorteilhafte ausgestaltungen sind in den Unterauspreichen enthalten. Die Vorrichtung ist eine Attrappe des diagnostischen und/oder chirurgischen Instrumentes. Es ist wenigstens eine Aufnahme zur Anordnung eines eine Testanschmutzung aufweisenden Prüfelementes in oder an einem Prüfkörper, der dem gehäuseförmigen Bauteil des Instrumentes entspricht und im Verlauf eines Prüfschlauches, der dem kanülenförmigen Bauteil entspricht, vorgesehen, wobei der gehäuseförmige Prüfkörper und der Prüfschlauch mittels einer Anschlussvorrichtung miteinander lösbar verbindbar sind.

Die Grundidee der Erfindung besteht also darin, dass die Vorrichtung einen Dummy des zu reinigenden/zu desinfizierenden diagnostischen und/oder chirurgischen Instrumentes darstellt, der an verschiedenen Stellen und Abschnitten im Gegensatz zum Originalinstrumerit aus voneinander lösbaren und schon damit leichter überprüfbaren Einzelelementen besteht und /oder mit einem eine Testanschmutzung aufweisenden Prüfelement versehen werden kann.

Dieser Dummy wird dann anstelle des echten Instrumentes in einem Waschautomaten einem Reinigungs- und Desinfektionsvorgang unterworfen. Auch anhand der Prüfelemente kann dann festgestellt werden, ob der Waschautomat in allen Teilen des Instrumentes Reinigungs- und Desinfektionsergebnisse erzielt, die den einschlägigen Hygiene- und Sicherheitsvorschriften entsprechen.

Wie vorher bereits erwähnt wird im Rahmen der vorliegenden Erfindung ein besonderes Augenmerk auf die Nachahmung eines Endoskopes gelegt, wobei das gehäuseförmige Bauteil ein Bedienteil und das kanülenförmige Bauteil ein Arbeitsteil des Endeskopes darstellt. Aufgrund dieser Bevorzugung befasst sich die weitere Darstellung der Erfindung im Wesentlichen mit einem Endoskop-Dummy. Durch die lösbare Verbindung des Prüfschlauches mit dem Prüfkörper kann des weiteren nach dem Waschvorgang durch das Entfernen des Prüfschlauches vom Prüfkörper die mit dem Prüfschlauch korrespondierende Eingangs- bzw. Ausgangsöffnung am Prüfkörper leichter kontrolliert werden.

Der gehäuseförmige Prüfkörperweist an seiner Umfangsfläche wenigstens eine außen liegende Aufnahmevorrichtung zum Befestigen wenigstens eines eine Testanschmutzung aufweisenden Prüfelementes auf. Dadurch wird eine sichere Befestigung des eine Testanschmutzung aufweisenden Prüfelementes an der Außenfläche des gehäuseförmigen Prüfkörpers erreicht.

Nach dem Stand der Technik wurde das Reinigungs- und Desinfektionsergebnis an der Außenfläche des Bedienteiles des Endoskops überprüft, indem man an der Umfangsfläche des Bedienteiles Prüfelemente, beispielsweise mittels Kabelbinder, befestigt hat. Mit der erfindungsgemäßen Aufnahmevorrichtung wird ein Verrutschen oder gar ein Ablösen der Prüfelemente vom Bedienelement des Endoskops verhindert, sodass nunmehr eine zuverlässige Aussage über das Reinigungs- und Desinfektionsergebnis getroffen werden kann.

Ein wesentliches Merkmal der Erfindung besteht darin, dass die Anschlussvorrichtung zum Verbinden des Prüfkörpers mit dem Prüfschlauch zur Aufnahme eines eine Testanschmutzung aufweisenden Prüfelementes ausgebildet ist. Bei dieser erfindungsgemäßen Vorrichtung kann also nach dem Waschvorgang der Prüfschlauch vom Prüfkörper getrennt werden und das Reinigungs- und Desinfektionsergebnis anhand des in der Aufnahmevorrichtung befindlichen Prüfelementes festgestellt werden. Somit kann im Übergangsbereich von Prüfkörper (Bedienteil) zu Prüfschlauch (Arbeitsteil), der beispielsweise bei einem einteiligen Endoskop eine kritische Stelle bezüglich anhaftender Kontaminierung darstellt, die Anschlussvorrichtung als erste Prüfkammer zur Aufnahme eines Prüfelementes dienen.

Mit der Erfindung ist es also erstmals möglich, Rückschlüsse auf das Reinigungs- und Desinfektionsergebnis auch des Bedienteiles eines Endoskops und damit auch auf das Reinigungs- und Desinfektionsergebnis eines einteiligen flexiblen Endoskops zu erhalten, wodurch das Risiko einer Infektion bei mehrmaliger Verwendung eines derartigen Endoskops erheblich minimiert werden kann.

Dabei spielt es keine wesentliche Rolle, ob die Anschlussvorrichtung von einer Schraubverbindung oder von einer Steckverbindung gebildet ist, vielmehr ist die Tatsache entscheidend, dass mit der neuartigen Vorrichtung das Reinigungs- und Desinfektionsergebnis der Übergangsstelle vom Bedienteil zum Arbeitsteil überprüft werden kann.

Weist der gehäuseförmige Prüfkörper, wie ein weiteres Ausführungsbeispiel der Erfindung vorsieht, einen dem Ventilkopf eines Endoskops entsprechenden Prüfventilkopf zum Einbringen von beispielsweise Reinigungsflüssigkeit in den Prüfschlauch und wenigstens eine weitere Anschlussvorrichtung zum Verbinden des Prüfkörpers mit einem weiteren Prüfschlauch, der vorzugsweise einem schlauchförmigen Versorgungsteil zum Einbringen von Druckluft entspricht, auf, wird eine noch praxisbezogenere Prüfvorrichtung für alle Arten von Endoskopen erreicht. Auch hier können die jeweiligen Anschlussteile zur Aufnahme eines Prüfelementes mit Testverschmutzungen ausgestaltet sein.

In bevorzugter Weise führt dabei der Transportkanal der weiteren Anschlussvorrichtung in eine vom Transportkanal des Prüfkörpers separierte Kammer, sodass mit einem an die Anschlussvorrichtung angeschlossenen Prüfschlauch, der dem Versorgungsteil eines Endoskopes zum Einbringen von Druckluft entspricht, die Funktionalität eines Dichtigkeitsprüfungsgerätes getestet werden kann.

An dem vom Prüfschlauch abgewandten Ende des Prüfkörpers ist anstelle einer Bildoptik des Endoskopes eine zweiteilig ausgestaltete erste Prüfkammer zur Aufnahme eines Prüfelementes dichtend einsetzbar. In diesem Bereich des Bedienteils eines Endokopes treffen senkrecht Transportkanäle, beispielsweise der eines Luer-Lock mit dem Optikkanal, aufeinander, sodass hier eine Überprüfung der Reinigungsleistung besonders wichtig ist.

Es ist daher des Weiteren von großem Vorteil, wenn die zweiteilig ausgestaltete Prüfkammer beabstandet zum Transportkanal einer solchen Anschlussvorrichtung zu liegen kommt. Da die Prüfkammer in diesem Fall nicht Bestandteil des eigentlichen Prüfkörpers ist, sondern in diesen eingeführt wird, ist die Prüfkammer mit Bohrungen versehen, damit ein darin angeordnetes Prüfelement zuverlässig von Flüssigkeit umspült werden kann.

Diese erste Prüfkammer ist in geeigneter Weise mittels Schraub-, Schnapp-, Bajonett- oder dergleichen Verschluss dichtend in dem Prüfkörper gehalten.

Im Verlauf des Prüfschlauches ist eine zweite Prüfkammer zur Aufnahme eines Prüfelementes integriert, welche vorzugsweise aus einer zylindrischen Hülse und einem darin unverlierbar und verschiebbar gehaltenen Stempel zur Aufnahme des Prüfelementes gebildet ist. Die Überprüfung der Reinigungsleistung ist hinsichtlich des Schlauchteiles von Endoskopen und derartiger Instrumente von besonderem Interesse, da diese Schlauchteile häufig nur sehr geringe Querschnitte, respektive Durchmesser von manchmal nur 1 bis 3 mm besitzen. Die Schlauchdurchmesser der erfindungsgemäßen Instrumenten-Dummys sind nicht beschränkt und können auf den jeweiligen fall angepasst werden.

Vorzugsweise sind zur einfachen Handhabung Hülse und Stempel dieser Prüfkammer mittels Schraub-, Schnapp-, Bajonett- oder dergleichen Verschluss dichtend arretierbar.

Zur Standardisierung der erfindungsgemäßen Vorrichtung sind die Aufnahmen zur Anordnung eines eine Testanschmutzung aufweisenden Prüfelementes auf Standardprüfelemente genormt.

Weitere Vorteile und Einzelheiten der Erfindung werden anhand der nachfolgenden Figurenbeschreibung unter Bezugnahme auf das in den Zeichnungen dargestellte Ausführungsbeispiel eines Endoskop-Dummys näher erläutert. Darin zeigt:
- Fig. 1a: schematisch ein Endoskop nach dem Stand der Technik,
- Fig. 1b: den Bedienteil aus Fig. 1a,
- Fig. 2a: ein Ausführungsbeispiel einer erfindungsgemäßen Vor- richtung mit einem Prüfkörper und einem Prüf- schlauch,
- Fig. 2b: ein zweites Ausführungsbeispiel der Erfindung,
- Fig. 3: einen Prüfkörper der erfindungsgemäßen Vorrichtung,
- Fig. 4: verschiedene Aufsätze für den Prüfventilkopf,
- Fig. 5a und 5b: einen Prüfkörper mit unterschiedlichen, am Prüfventilkopf angeordneten Aufsätzen und
- Fig. 6: das Ausführungsbeispiel gemäß Fig. 2a in zerlegtem Zustand,
- Fig. 7: eine genauere Darstellung einer ersten Prüfkammer,
- Fig. 8: eine genauere Darstellung einer zweiten Prüfkammer und
- Fig. 9: eine genauere Darstellung einer dritten Prüfkammer.

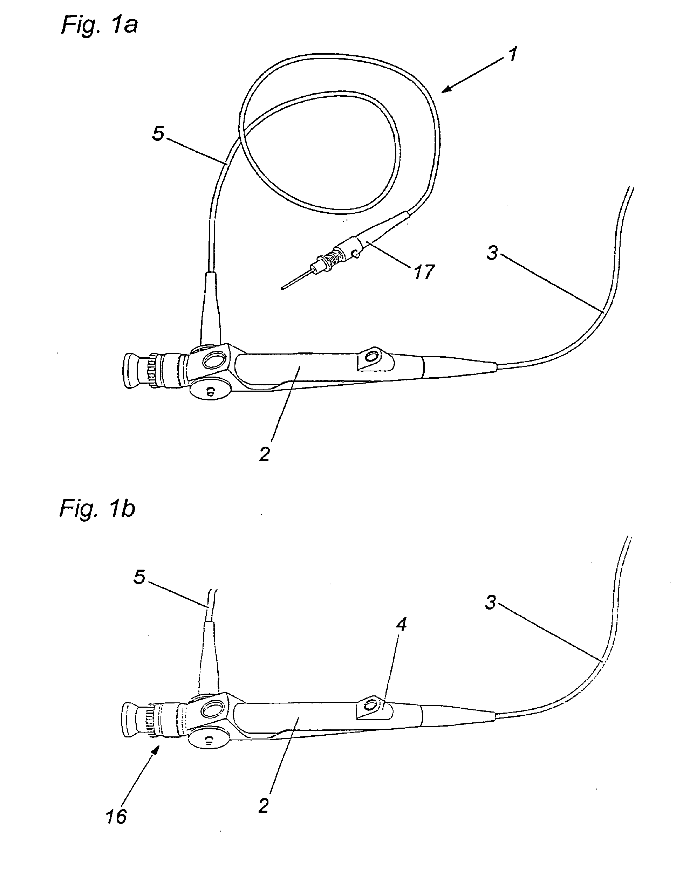

In den Fig. 1a und 1b ist ein einteiliges flexibles Endoskop 1 dargestellt, bei dem der schlauchförmige Arbeitsteil 3 unlösbar mit dem Bedienteil 2 verbunden ist. An einer Anschlussvorrichtung des Bedienteiles 2 ist ein Versorgungsteil 5 angeordnet, an dessen freiem Ende ein Ventil 17 zum

Einbringen von Druckluft angeordnet ist. Wie insbesondere aus Fig. 1b ersichtlich, weist das Bedienteil 2 eines Endoskops 1 einen Ventilkopf 4 auf, über den beispielsweise medizinische Instrumente in den Arbeitsteil 3 des Endoskops 1 eingebracht werden können. Zudem ist im Bedienteil 2 die Bildoptik 16 angeordnet, die mit der am freien Ende des Arbeitsteiles 3 angeordneten Sonde über im Arbeitsteil 3 verlaufende Glasfaserbündel verbunden ist.

Diese Glasfaserbündel machen eine Trennung des Arbeitsteiles 3 vom Bedienteil 2 für Reinigungszwecke unmöglich, weshalb man, wie bereits erwähnt, Prüfkörper, die zur Aufnahme eines eine Testanschmutzung aufweisenden Prüfelementes ausgebildet sind, entwickelt hat.

Mit diesen Prüfkörpern, die, obwohl schlauchförmig, das Arbeitsteil eines Endoskopes nicht simulieren können, lässt sich eine mehr oder weniger zutreffende Aussage darüber machen, ob der Arbeitsteil 3 eines Endoskops 1 beim Waschvorgang den Anforderungen entsprechend gereinigt wird oder nicht. Ob aber das vollständige Instrument 1 und insbesondere das Bedienteil 2 bzw. die Übergangsstelle vom Bedienteil 2 zum Arbeitsteil 3 ordnungsgemäß gereinigt wird, lässt sich mit derartigen Prüfkörpern alleine nicht feststellen, auch ist die Sicherheit der Überprüfung für den Arbeitsteil nicht vollständig gegeben.

Hier setzt die Erfindung an und stellt eine Vorrichtung 6 zur Überprüfung des Reinigungs- und Desinfektionsergebnisses bei in Waschautomaten gereinigten diagnostischen und/oder chirurgischen Instrumenten 1 zur Verfügung.

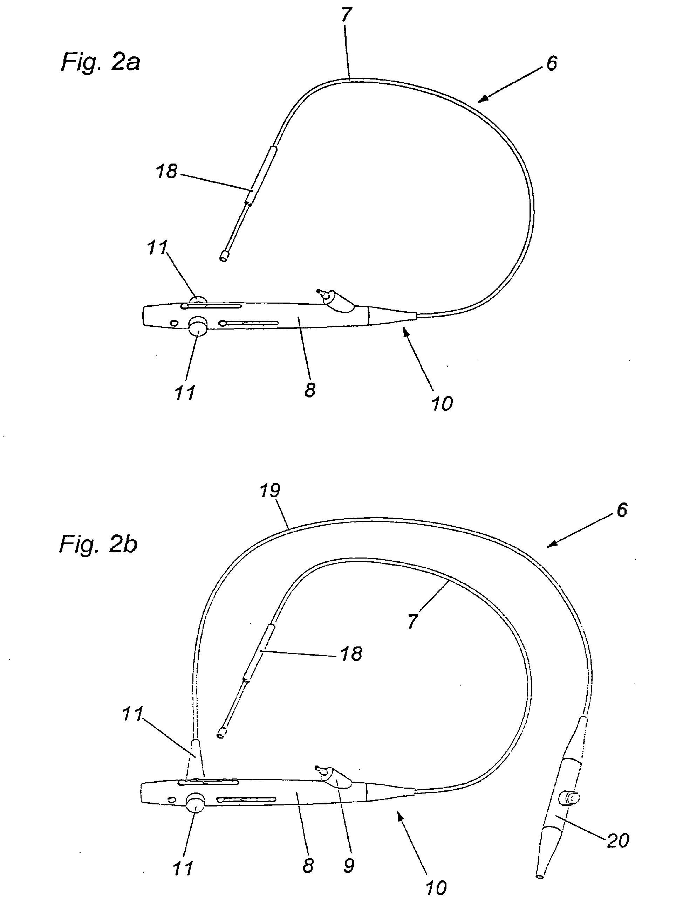

Die erfindungsgemäße Vorrichtung 6 weist neben einem Prüfschlauch 7 weiters einen Prüfkörper 8, der dem gehäuseförmigen Bedienteil 2 eines Endoskops 1 entspricht, auf, wie dies in Fig. 2a dargestellt ist. Im Verlauf des Prüfschlauches 7 ist dabei eine zweite Prüfkammer 18 angeordnet, die zur Aufnahme eines Prüfelementes 14 dient und wird weiter unten genauer beschrieben. Der Prüfschlauch 7 ist mit dem Prüfkörper 8 über eine Anschlussvorrichtung 10 lösbar verbunden. Zudem weist der Prüfkörper 8 des gezeigten Ausführungsbeispieles zwei weitere Anschlussvorrichtungen 11 zum Verbinden des Prüfkörpers 8 mit weiteren Prüfschläuchen auf.

Ein solcher weiterer Prüfschlauch 19 ist bei dem in Fig. 2b gezeigten Ausführungsbeispiel über die Anschlussvorrichtung 11 mit dem Prüfkörper 8 verbunden. Der Prüfschlauch 19 entspricht dabei dem in Fig. 1a dargestellten Versorgungsteil 5 des Endoskops 1, wobei am dem Prüfkörper 8 abgewandten Ende des Prüfschlauches 19 ein Prüfventil 20 zum Einbringen von Druckluft angeordnet ist. Diese Maßnahme wird zur Überprüfung der Dichtigkeit der Vorrichtung bei einem vom Waschautomaten-Hersteller angegebenen Betriebsdruck verwendet.

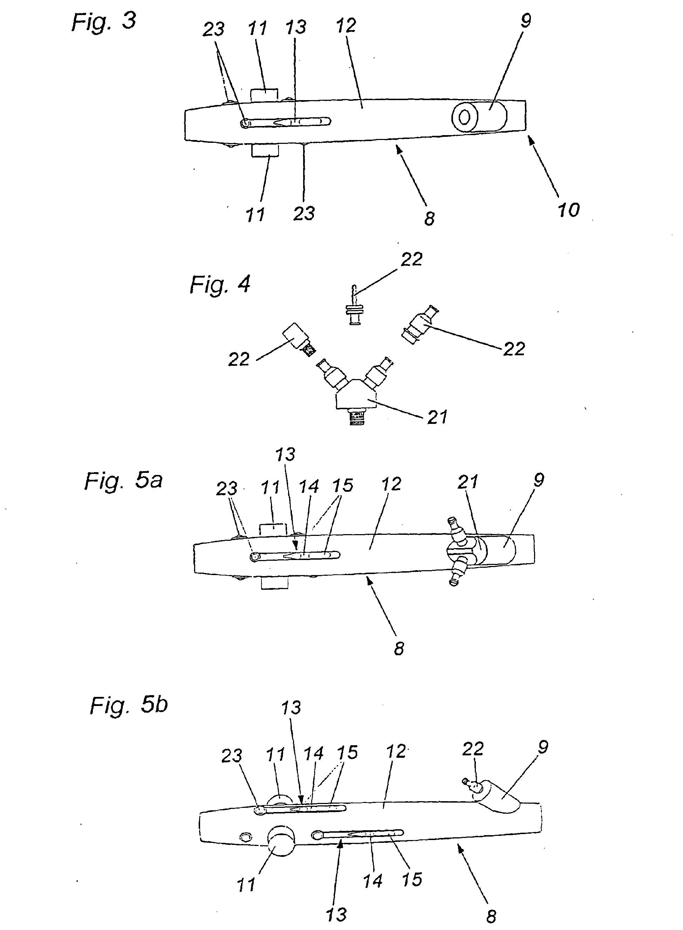

Neben den Anschlussvorrichtungen 10 und 11 weist der Prüfkörper 8 zudem einen Prüfventilkopf 9 sowie mehrere Aufnahmevorrichtungen 13 auf, die an der Umfangsfläche 12 in Form von nutenförmigen Ausfräsungen bzw. Taschen ausgebildet sind (Fig. 3) und in welchen die Prüfelemente 14 unverlierbar gehalten werden. Gegebenenfalls können die Prüfelemente 14 über Befestigungsmittel 23 in den Aufnahmebereichen 13 befestigt werden. Auf dem Prüfelement 14 sind Testanschmutzungen 15 aufgebracht, die beispielsweise synthetisch hergestellt oder von frischem Human-, Rinderzitrat- oder Hammelblut gebildet sein können (Fig. 5a, 5b).

Auf dem Prüfventilkopf 9 können unterschiedliche Aufsätze 21, 22 angeordnet werden, die beispielsweise dem Einbringen von Wasser oder Druckluft dienen. Eine Besonderheit stellt in diesem Zusammenhang der Aufsatz 21 dar, der in Form eines T-Stückes ausgebildet ist und dem gleichzeitigen oder alternativen Einbringen einer Sonde und/oder Wasser und/oder Reinigungsflüssigkeit in den Prüfventilkopf 9 dient (Fig. 4).

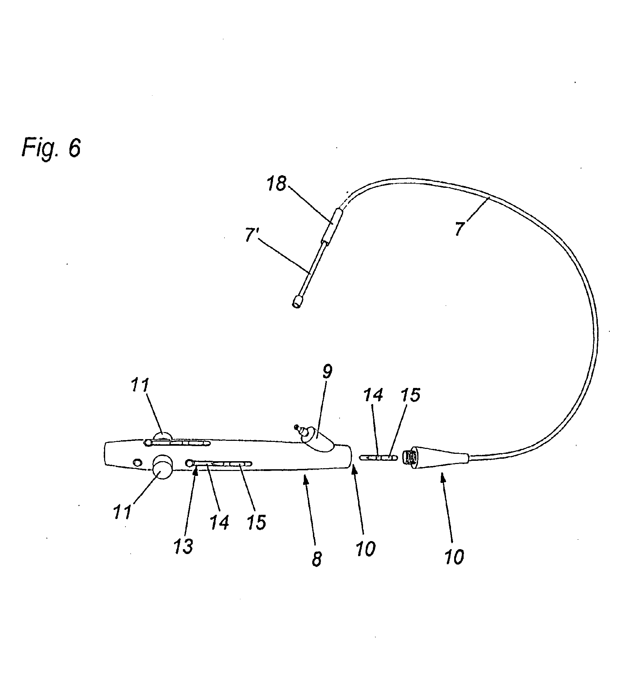

In der Fig. 6 ist das Ausführungsbeispiel der Erfindung gemäß Fig. 2a in einem Zustand dargestellt, in welchem der Prüfschlauch 7 von dem Prüfkörper 8 getrennt ist. Das Anschlussteil 10 und der Übergang 10' zum Prüfkörper sind so ausgestaltet, dass darin ein Prüfelement 14 mit Testverschmutzungen 15 angeordnet werden kann. Das Anschlussteil 10 bildet damit zusammen mit dem Übergang 10' eine erste Prüfkammer für einen kritischen Bereich innerhalb eines Endoskopes, wo der Biopsiekanal des Bedienteils auf den flexiblen Arbeitsteil eines Endoskopes trifft.

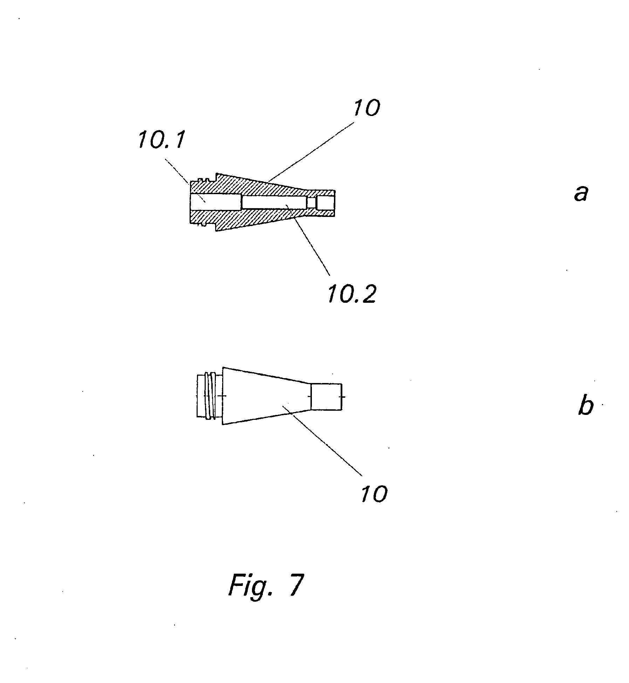

In Fig. 7 ist eine beispielhafte Ausgestaltung eines solchen Anschlussteiles 10 im Querschnitt (a) und in Seitenansicht (b)gezeigt. Der Innenraum ist in zwei Bereiche 10.1 und 10.2 unterteilt, was das Anschlussteil 10 zur Aufnahmen von Prüfelementen unterschiedlicher Abmessungen geeignet macht. Beispielsweise würde ein Prüfelement von 5x56x1 (mm) beide Bereiche 10.1 und 10.2 durchgreifen, wohingegen ein Prüfelement mit 6,5x30x0,8 (mm) nur in den Bereich 10.2 eindringen würde. Es ist festzuhalten, dass das Prüfelement 14 nicht ganz von dem Anschlussteil 10 aufgenommen wird, sondern etwa zur Hälfte in den Übergang 10' des Prüfkörpers 8 (vgl. Fig. 6) hineinragt, wenn Prüfkörper 8 und Prüfschlauch 7 miteinander verbunden sind.

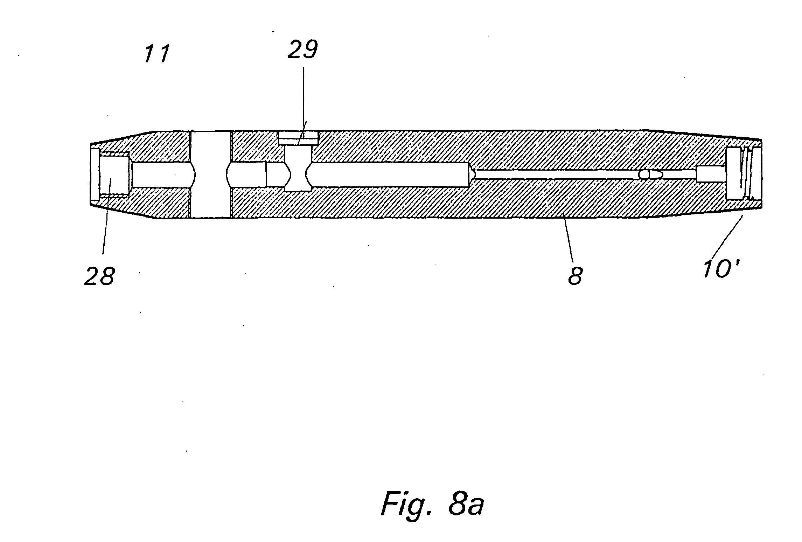

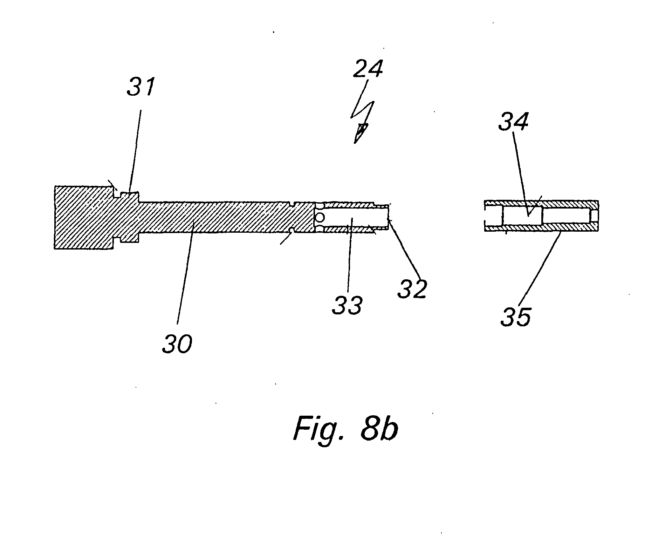

Fig. 8 zeigt Anbringung und Ausgestaltung einer ersten Prüfkammer 24, die an dem vom Prüfschlauch abgewandten Ende des Prüfkörpers 8 dichtend einsetzbar ist. Hierbei ist in Fig. 8a schematisch ein Schnitt durch einen gesamten Prüfkörper 8 gezeigt, in dem insbesondere der Verlauf und die Anordnung der Wasser-, Luft- und Biopsiekanäle eines Endoskopes nachgebildet sind. Am linken Ende ist eine Aufnahme bzw. Öffnung 28 vorgesehen, durch welche die erste Prüfkammer 24 (Fig. 8b) eingeführt und dicht mit dem Prüfkörper 8 verbunden werden kann. Die erste Prufkammer 24 muss etwa in Höhe der Öffnung 29 zu liegen kommen, bei der es sich um den Zugang zu einem Wasser-/Luftkanal eines Endoskopes handelt und bei dem Dummy als Spülmittelzuführung dient.

Wie aus Fig. 8b ersichtlich ist, ist die erste Prüfkammer 24 zweiteilig ausgestaltet. Dazu wird ein verlängerter Stempel 30, der mittels Schraub-, Schnapp-, Bajonett- oder dergleichen Verbindung 31 mit dem Prüfkörper 8 dicht verbunden werden kann, an dem der Verbindungsstelle abgewandten Ende 36 hohl ausgeführt und mit einem beispielsweise Schraubgewinde 32 versehen. Dieser hohle Bereich bildet einen ersten Raumteil 33 des ersten Prüfkörpers 24. Der zweite Raumteil 34 wird von einer Hülse 35 gebildet, die mit dem ersten Raumteil 33 verbunden werden kann. In dem so ausgebildeten Raum kann ein Prüfelement 14 unverlierbar gehalten werden, indem der Hülseninnendurchmesser am Ende der Hülse 35 geringer ist wie an der Verbindungstelle zum ersten Raumteil 33. Im Bereich des ersten Raumteils 33 sind Bohrungen bzw. Löcher in die Prüfkammer eingebohrt, damit eine Reinigunsflüssigkeit über ein eingesetztes Prüfelement 14 strömen kann. Der Innendurchmesser der Hülse 35 verjüngt sich wie gezeigt in Stufen, so dass wieder Prüfelemente 14 verschiedener Abmessungen aufgenommen werden können, wie bereits für die erste Prüfkammer des Anschlussteiles 10 beschrieben worden ist.

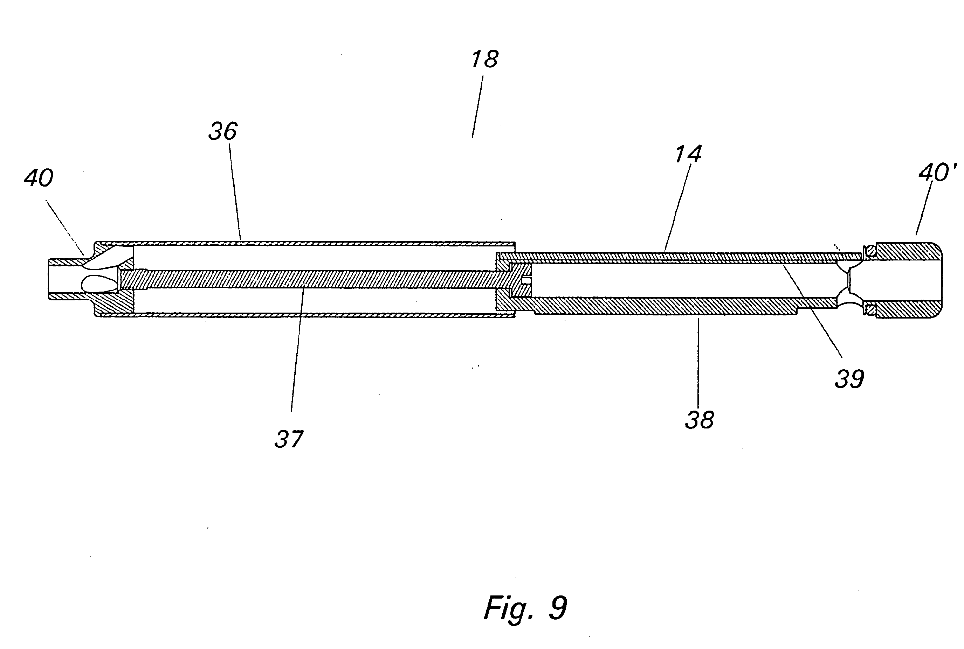

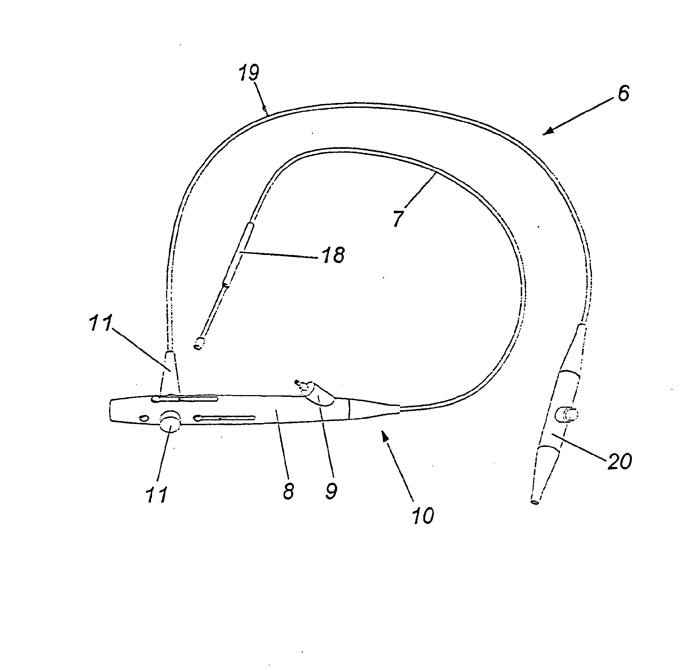

Schließlich ist noch in Fig. 9 eine zweite Prüfkammer 18 skizziert, wie sie erfindungsgemäß im Prüfschlauch 7 angeordnet ist. Diese Prüfkammer 18 lässt sich öffnen und schließen, beispielsweise wieder mit einer Schraub-, Schnapp-, Bajonett-oder dergleichen Verbindung, ist aber nicht trennbar. Dazu weist eine Hülse 36 in ihrem Inneren mittig eine Stange 37 auf, über welche eine zweite Hülse 38 geführt ist, derart, dass im ausgezogenen Zustand die Hülse 38 mit der Stange verankert ist. Die Hülse 38 weist einen abgeflachten Bereich 39 auf, auf dem das Prüfelement 14 gelagert werden kann. Beide Enden 40 und 40' sind mit dem Prüfschlauch 7 verbunden und die Kammer wird während eines Testreinigungsganges von Flüssigkeit durchströmt.

Die Erfindung stellt durch die Anordnungsmöglichkeit von Prüfelementen 14 mit einer oder mehreren Testanschmutzungen 15 im Verlauf des Prüfschlauches 7, in der Übergangsstelle vom Prüfschlauch 7 zum Prüfkörper 8, im Bereich des Wasser-/Luftkanals und an der Umfangsfläche 12 des Prüfkörpers 8 eine gegenüber dem Stand der Technik stark verbesserte und zuverlässige Überprüfungsmöglichkeit des Reinigungs- und Desinfektionsergebnisses von in Waschautomaten gereinigten Instrumenten, insbesondere Endoskopen dar. Auch die weiteren Testschläuche 19, welche einen Versorgungsschlauch 5 simulieren, können mit diesen Merkmalen ausgestattet sein.

Wenn auch die Erfindung anhand der gezeigten Ausführungsbeispiele im Detail beschrieben wurde, versteht es sich von selbst, dass die Erfindung nicht auf die dargestellten Ausführungsbeispiele beschränkt ist. Vielmehr sind Modifikationen und Abwandlungen durchaus erwünscht und möglich. So könnten beispielsweise anstelle der Schraubverbindungen Steckverbindungen zum Einsatz kommen. Aber auch die Verwendung anderer als in dem Ausführungsbeispiel dargestellter Prüfelemente mit Testanschmutzungen wird von der Erfindungsidee umfasst. Auch mehrere Prüfschläuche und -kanäle können vorhanden sein.

## Patentansprüche

1. Vorrichtung (6) zur Überprüfung des Reinigungs- und Desinfektionsergebnisses bei insbesondere in Waschautomaten gereinigten diagnostischen und/oder chirurgischen Instrumenten mit gehäuseförmigem Bedienteil (2) und kanülenförmigem Arbeitsteil (3), wobei die Vorrichtung (6) als Attrappe des diagnostischen und/oder chirurgischen Instrumentes mit einem dem Bedienteil (2) des Instrumentes entsprechenden Prüfkörper (8) und einem dem Arbeitsteil (3) des Instrumentes entsprechenden Prüfschlauch (7) ausgebildet ist, wobei der Prüfkörper (8) und der Prüfschlauch (7) mittels einer Anschlussvorrichtung (10) miteinander lösbar verbindbar sind, wobei die Anschlussvorrichtung (10) zur Aufnahme eines Prüfelementes (14) mit Testanschmutzung (15) ausgebildet ist,
wobei der Prüfkörper (8) zur Anordnung eines Prüfelementes (14) mit einer Testanschmutzung (15) an seiner Umfangsfläche (12) wenigstens eine außen liegende Aufnahmevorrichtung (13) und eine innen liegende zweiteilig ausgestaltete erste Prüfkammer (24) ist,
und wobei im Verlauf des Prüfschlauchs (7) eine zweite Prüfkammer (18) ebenfalls zur Anordnung eines Prüfelementes (14) mit einer Testanschmutzung (15) vorgesehen ist, die zweite Prüfkammer (18) unteilbar ausgestaltet ist und die erste Prüfkammer (24) an dem vom Prüfschlauch (7) abgewandten Ende des Prüfkörpers (8) einsetzbar und dicht verbindbar ist.

2. Vorrichtung (6) nach Anspruch 1, **dadurch gekennzeichnet, dass** die Vorrichtung eine Endoskop-Attrappe ist.

3. Vorrichtung (6) nach Anspruch 1, **dadurch gekennzeichnet, dass** die Anschlussvorrichtung (10) von einer Schraubverbindung gebildet ist.

4. Vorrichtung (6) nach Anspruch 1, **dadurch gekennzeichnet, dass** die Anschlussvorrichtung (10) von einer Steckverbindung gebildet ist.

5. Vorrichtung (6) nach einem der Ansprüche 2 bis 4, **dadurch gekennzeichnet, dass** der Prüfkörper (8) einen dem Ventilkopf (4) eines Endoskops entsprechenden Prüfventilkopf (9) zum Einbringen von beispielsweise Reinigungsflüssigkeit in des Prüfschlauch (7) aufweist.

6. Vorrichtung (6) nach einem der Ansprüche 1 bis 5, **dadurch gekennzeichnet, dass** der Prüfkörper (8) wenigstens eine weitere Anschlussvorrichtung (11) zum Verbinden mit einem weiteren Prüfschlauch (19), der vorzugsweise einem schlauchförmigen Versorgungsteil (5) zum Einbringen von Druckluft entspricht, aufweist.

7. Vorrichtung (6) nach Anspruch 6, **dadurch gekennzeichnet, dass** ein Transportkanal der Anschlussvorrichtung(en) (11) in eine von einem Transportkanal des Prüfkörpers (8) separierte Kammer führt.

8. Vorrichtung (6) nach Anspruch 1, **dadurch gekennzeichnet, dass** die erste Prüfkammer (24) beabstandet zum Transportkanal der Anschlussvorrichtung(en) (11) liegt und Bohrungen (25) aufweist.

9. Vorrichtung (6) nach Anspruch 8, **dadurch gekennzeichnet, dass** die erste Prüfkammer (24) in dem Prüfkörper (8) mittels Schraub-, Schnapp- oder Bajonettverbindung einsetzbar ist.

10. Vorrichtung nach Anspruch 1, **dadurch gekennzeichnet, dass** die zweite Prüfkammer (18) aus einer Hülse (26) und einem darin unverlierbar und verschiebbar gehaltenen Stempel (27) zur Auflage eines Prüfelementes (14) gebildet ist.

11. Vorrichtung nach einem der vorhergehenden Ansprüche 1 bis 10, **dadurch gekennzeichnet, dass** die Aufnahmevorrichtungen und Prüfkammern zur Anordnung eines Prüfelementes (14) mit Testanschmutzung (15) auf Standardprüfelemente genormt sind.

## Claims

1. Device (6) for verifying the cleaning and disinfection results of diagnostic and/or surgical instruments, in particular when cleaned in automatic washing devices, with a housing-shaped operating part (2) and tubular working part (3), with the device (6) being shaped as a mock-up of the diagnostic and/or surgical instrument with a test probe (8) matching the instrument's operating part (2) and a test tube (7) matching the instrument's working part (3), with the test probe (8) and the test tube (7) being joinable via a connection device (10) in a detachable fashion, with the connection device (10) being shaped for receiving a test element (14) with a test contamination (15),
with the test probe (8) featuring at least one exterior receiving device (13) for mounting a test element (14) with a test contamination (15) on its circumference surface (12) and one interior first test chamber (24) shaped in two parts, and
with a second test chamber (18) provided along the length of the test tube (7) also for fitting a test element (14) with a test contamination (15), the second test chamber (18) being shaped undivisible in one part and the first test chamber (24) being insertable at the test probe's (8) end opposite the test tube (7) and joinable in a tight-sealed fashion.

2. Device (6) in accordance with claim 1, **characterized in that** the device is an endoscope mock-up.

3. Device (6) in accordance with claim 1, **characterized in that** the connection device (10) is made up of a screw connection.

4. Device (6) in accordance with claim 1, **characterized in that** the connection device (10) is made up of a plug connection.

5. Device (6) in accordance with any of the claims 2 to 4, **characterized in that** the test probe (8) features a test valve head (9) matching the valve head (4) of an endoscope for inducing, for example, cleaning fluid into the test tube (7).

6. Device (6) in accordance with any of the claims 1 to 5, **characterized in that** the test probe (8) features at least one additional connection device (11) for attaching another test tube (19) matching preferably a tubular supply unit (5) for inducing compressed air.

7. Device (6) in accordance with claim 6, **characterized in that** one transport duct of the connection device (s) (11) leads into a chamber separated from a transport duct of the test probe (8).

8. Device (6) in accordance with claim 1, **characterized in that** the first test chamber (24) is located at a distance to the transport duct of the connection device (s) (11) and features bores (25).

9. Device (6) in accordance with claim 8, **characterized in that** the first test chamber (24) can be inserted into the test probe (8) via screw, snap or bayonet connection.

10. Device in accordance with claim 1, **characterized in that** the second test chamber (18) is designed as a sleeve (26) and a stamp (27) contained within in a captive and adjustable fashion as a basis for a test element (14).

11. Device in accordance with any of the above claims 1 to 10, **characterized in that** the receiving devices and test chambers for mounting a test element (14) with a test contamination (15) are standardised on standard test elements.

## Revendications

1. Dispositif (6) pour la vérification du résultat de nettoyage et de désinfection dans des instruments de diagnostic et/ou chirurgicaux nettoyés en particulier dans des appareils de lavage automatiques et comportant une partie de manipulation (2) en forme de boîtier et une partie de travail (3) en forme de canule, le dispositif (16) étant réalisé sous forme d'imitation de l'instrument de diagnostic et/ou chirurgical avec un corps de vérification (8) correspondant à la partie de manipulation (2) de l'instrument et un tuyau de vérification (7) correspondant à la partie de travail (3) de l'instrument, tels que le corps de vérification (8) et le tuyau de vérification (7) sont susceptibles d'être reliés l'un à l'autre de façon détachable au moyen d'un dispositif de raccordement (10), ledit dispositif de raccordement (10) étant réalisé en vue de la réception d'un élément de vérification (14) avec une salissure test (15),
dans lequel le corps de vérification (8) comporte, pour l'agencement d'un élément de vérification (14) avec une salissure test (15), au moins un dispositif récepteur (13) disposé à l'extérieur sur sa surface périphérique (12) et une première chambre de vérification (24) disposée à l'intérieur et conçue en deux parties,
et dans lequel dans le parcours du tuyau de vérification (7) est prévue une seconde chambre de vérification (18) également en vue de l'agencement d'un élément de vérification (14) avec une salissure test (15), ladite seconde chambre de vérification (18) étant conçue de façon non divisible et la première chambre de vérification (24) étant susceptible d'être mise en place et reliée de façon étanche à l'extrémité du corps de vérification (8) détournée du tuyau de vérification (7).

2. Dispositif (6) selon la revendication 1, **caractérisé en ce que** le dispositif est une imitation d'endoscope.

3. Dispositif (6) selon la revendication 1, **caractérisé en ce que** le dispositif de raccordement (10) est formé par une liaison à vis.

4. Dispositif (6) selon la revendication 1, **caractérisé en ce que** le dispositif de raccordement (10) est formé par une liaison à enfichage.

5. Dispositif (6) selon l'une des revendications 2 à 4, **caractérisé en ce que** le corps de vérification (8) comporte une tête avec valve de vérification (9), correspondant à la tête de valve (4) d'un endoscope pour l'introduction par exemple de liquide de nettoyage dans le tuyau de vérification (7).

6. Dispositif (6) selon l'une des revendications 1 à 5, **caractérisé en ce que** le corps de vérification (8) comporte au moins un second dispositif de raccordement (11) pour la liaison avec un autre tuyau de vérification (19), qui correspond de préférence à une partie d'alimentation (5) en forme de tuyau pour l'introduction d'air comprimé.

7. Dispositif (6) selon la revendication 6, **caractérisé en ce qu'**un canal de transport du/des dispositif(s) de raccordement (11) mène dans une chambre séparée d'un canal de transport du corps de vérification (8).

8. Dispositif (6) selon la revendication 1, **caractérisé en ce que** la première chambre de vérification (24) est disposée à distance du canal de transport du/des dispositif(s) de raccordement (11) et comporte des perçages (25).

9. Dispositif (6) selon la revendication 8, **caractérisé en ce que** la première chambre de vérification (24) est susceptible d'être mise en place dans le corps de vérification (8) au moyen d'une liaison à vis, à encliquetage ou à baïonnette.

10. Dispositif selon la revendication 1, **caractérisé en ce que** la seconde chambre de vérification (18) est formée par une douille (26) et par un poussoir (27), maintenu dans celle-ci de façon imperdable et déplaçable, pour la pose d'un élément de vérification (14).

11. Dispositif selon l'une des revendications 1 à 10, **caractérisé en ce que** les dispositifs de réception et des chambres de vérification pour l'agencement d'un élément de vérification (14) avec salissure test (15) sont normalisés à des éléments de vérification standard.
